# EUROPEAN PATENT APPLICATION

(11) **EP 4 434 374 A1**
(43) Date of publication of application: **25.09.2024**
(21) Application number: 21964768.2
(22) Date of filing: 19.11.2021
(51) Int. Cl.: A24F 40/57

(54) **SUCTION DEVICE**

(71) Applicant: Japan Tobacco Inc., Tokyo 105-6927 (JP)
(72) Inventor: NAKANO, Takuma, Tokyo 130-8603 (JP); MIZUGUCHI, Kazuma, Tokyo 130-8603 (JP)
(74) Representative: Hoffmann Eitle
(86) International application number: PCT/JP2021/042541
(87) International publication number: WO 2023/089753

(57) **Abstract**

A inhalation device comprising: a liquid storage part that stores a liquid for generating an aerosol by being heated; a heating part that heats the liquid; a power source part that stores power; and a control part that controls the supply of power from the power source part to the heating part, wherein the control part controls the supply of power for performing first heating to set the temperature of the liquid to a first temperature or higher for vaporizing the liquid when a previously determined prescribed condition is established, performs second heating by supplying power so that the temperature of the liquid becomes a second temperature or higher and a temperature lower than the first temperature before the prescribed condition is established, and thereafter stops the second heating when a previously determined finishing condition is established.

## Description

### Technical Field

The present invention relates to an inhalation device.

### Background Art

In recent years, techniques have been proposed for rapidly providing aerosol when a user inhales.

For example, an apparatus described in PTL 1 includes a heater that generates aerosol by heating an aerosol source, and a controller that can change the amount of electric power supplied to the heater in order to heat the aerosol source at a preheating temperature lower than a heating temperature for generating aerosol.

### Citation List

### Patent Literature

PTL 1: U.S. Patent Application Publication No. 2020/0329776

### Summary of Invention

### Technical Problem

In the technique described in PTL 1, regardless of whether a user performs an inhaling operation, between inhaling operations by the user, preliminary heating is continued to heat an aerosol source at a preheating temperature lower than a heating temperature for generating aerosol. Thus, if an inhaling operation is not performed after the preliminary heating is performed, electric power consumption for the preliminary heating is wasted. When performing heating for setting a temperature lower than the temperature at which aerosol is generated, it is desirable to suppress wasteful electric power consumption associated with the heating.

An object of the present invention is to provide an inhalation device capable of suppressing wasteful electric power consumption associated with heating for setting a temperature lower than the temperature at which aerosol is generated. Solution to Problem

A first feature of the present invention completed under such an object is an inhalation device including: a liquid storage configured to store a liquid for generating aerosol by being heated; a heater configured to heat the liquid; a power supply configured to accumulate electric power; and a controller configured to control electric power supply from the power supply to the heater, in which, if a predetermined condition determined in advance is satisfied, the controller controls the electric power supply to perform first heating in which a temperature of the liquid becomes higher than or equal to a first temperature at which the liquid is vaporized, and, before the predetermined condition is satisfied, the controller performs, by performing the electric power supply, second heating in which the temperature of the liquid becomes a temperature higher than or equal to a second temperature and lower than the first temperature, and then, if an end condition determined in advance is satisfied, the controller stops the second heating.

A second feature may be that the end condition is satisfied if a time determined in advance elapses after the second heating is started.

A third feature may be that a gyro sensor is provided, and the end condition is satisfied if an output value of the gyro sensor changes, from a value indicating that a height of an inhalation port to be held in a mouth at a time of inhalation of the aerosol is higher than a height of the power supply, to a value indicating that the power supply and the inhalation port are at the same height.

A fourth feature may be that an acceleration sensor is provided, and the end condition is satisfied if an output value of the acceleration sensor becomes smaller than or equal to a negative threshold value determined in advance.

A fifth feature may be that a tactile sensor is provided, and the end condition is satisfied if an output value of the tactile sensor does not indicate a value indicating a contact of a hand of a user.

A sixth feature may be that the end condition is satisfied if a distance to a mouth of a user exceeds a threshold value determined in advance.

A seventh feature may be that at least one device of a LiDAR configured to measure the distance, an infrared sensor configured to measure a body temperature of the user, a camera, an odor sensor, a tactile sensor, a humidity sensor, and a CO₂ sensor is provided, and the controller grasps that the distance exceeds the threshold value by using an output value of the device.

An eighth feature may be that the controller sets a value of the electric power at a time of the second heating to be smaller than a value the electric power at a time of the first heating.

A ninth feature may be that the controller performs control such that a temperature of the heater does not exceed a target temperature.

### Advantageous Effects of Invention

According to the first feature, since the second heating is stopped if it becomes unnecessary to perform the second heating for setting a temperature lower than the temperature at which aerosol is generated, it is possible to suppress wasteful electric power consumption associated with the second heating.

According to the second feature, since the second heating can be stopped with higher accuracy, it is possible to suppress wasteful electric power consumption associated with the second heating, with higher accuracy.

According to the third feature, it is possible to detect that it becomes unnecessary to perform the second heating, with higher accuracy.

According to the fourth feature, it is possible to detect that it becomes unnecessary to perform the second heating, with higher accuracy.

According to the fifth feature, it is possible to detect that it becomes unnecessary to perform the second heating, with higher accuracy.

According to the sixth feature, it is possible to detect that it is unnecessary to perform the second heating, with higher accuracy.

According to the seventh feature, it is possible to determine that the distance to the mouth of the user exceeds the threshold value, with higher accuracy.

According to the eighth feature, even if the inhaling operation is not performed after the second heating is performed, it is possible to suppress wasteful electric power consumption.

According to the ninth feature, since the electric power is not supplied more than necessary, it is possible to suppress electric power consumption at the time of the second heating.

### Brief Description of Drawings

[Fig. 1] Fig. 1 is an example of a perspective view illustrating a schematic configuration of an inhalation device.
[Fig. 2] Fig. 2 is an example of a cross-sectional view illustrating a schematic configuration of the inhalation device.
[Fig. 3] Fig. 3 is a diagram schematically illustrating an example of a schematic configuration of the inhalation device.
[Fig. 4] Fig. 4 is a flowchart illustrating an example of a procedure of a heating process performed by a controller.
[Figs. 5A to 5C] Figs. 5A to 5C are timing charts for describing operations of the inhalation device.
[Fig. 6] Fig. 6 is a diagram illustrating an example of a schematic configuration of a sensor and a controller according to modifications.
[Fig. 7] Fig. 7 is a diagram schematically illustrating an example of a schematic configuration of an inhalation device according to a second embodiment.
[Fig. 8] Fig. 8 is a diagram schematically illustrating an example of a schematic configuration of an inhalation device according to a third embodiment.
[Fig. 9] Fig. 9 is a diagram schematically illustrating an example of a configuration of an inhalation device according to a fourth embodiment.
[Figs. 10A to 10C] Figs. 10A to 10C are timing charts for describing operations of the inhalation device according to the fourth embodiment.

### Description of Embodiments

Hereinafter, embodiments of the present invention will be described in detail with reference to the accompanying drawings.

### <First Embodiment>

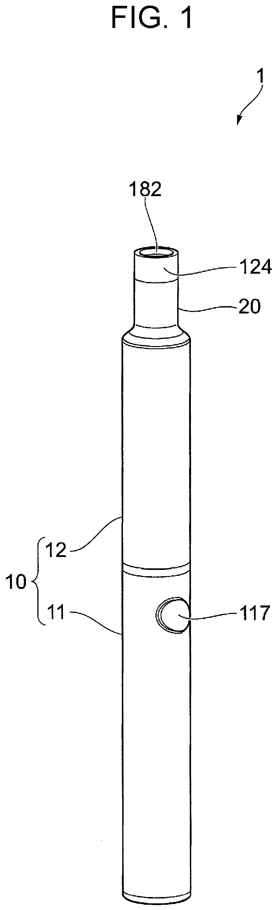

Fig. 1 is an example of a perspective view illustrating a schematic configuration of an inhalation device 1.

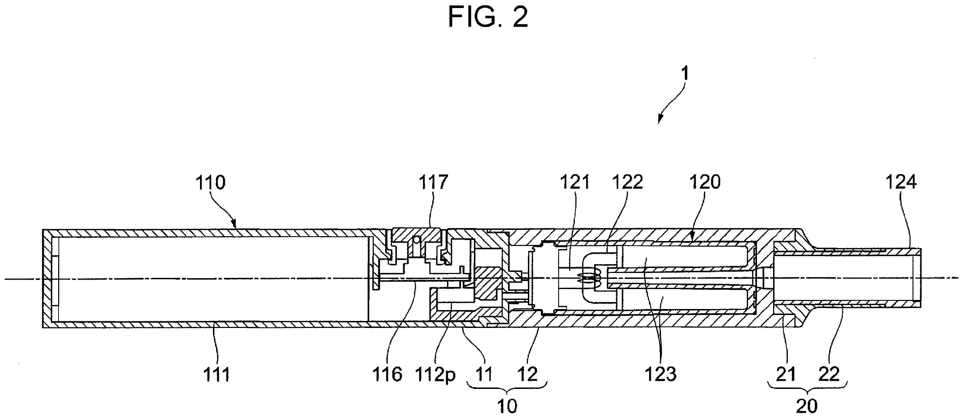

Fig. 2 is an example of a cross-sectional view illustrating a schematic configuration of the inhalation device 1.

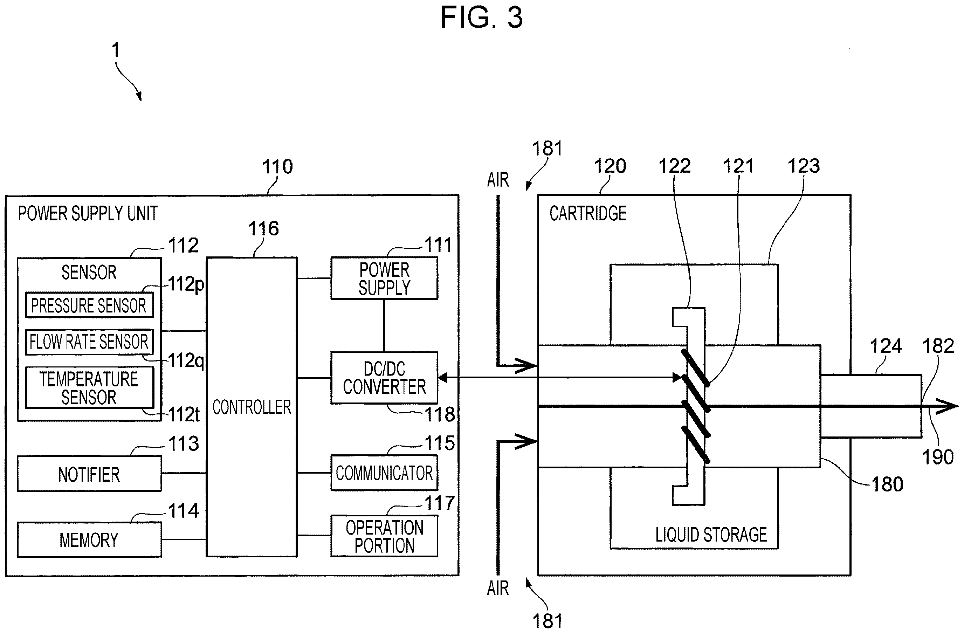

Fig. 3 is a diagram schematically illustrating an example of a schematic configuration of the inhalation device 1.

The inhalation device 1 according to the first embodiment is a device that generates a substance to be inhaled by a user. In the following description, the substance generated by the inhalation device 1 is aerosol. Alternatively, the substance generated by the inhalation device 1 may be gas.

The inhalation device 1 generates aerosol by heating an aerosol source as a liquid. The inhalation device 1 includes a power supply unit 110, a cartridge 120, a case 10 that accommodates the power supply unit 110 and the cartridge 120, a mouthpiece 124, and an end cap 20 that accommodates a part of the mouthpiece 124. The power supply unit 110 and the cartridge 120 are configured to be attachable to and detachable from each other. The user inhales in a state in which the cartridge 120 is attached to the power supply unit 110.

As illustrated in Fig. 3, the power supply unit 110 includes a power supply 111, a sensor 112, a notifier 113, a memory 114, a communicator 115, and a controller 116. The power supply unit 110 includes an operation portion 117 operable by the user, and a DC/DC converter 118. The cartridge 120 includes a heater 121, a liquid guide 122, and a liquid storage 123. An airflow path 180 is formed in the inhalation device 1. Hereinafter, each structural element will be described in order

### (Power Supply Unit 110)

The power supply 111 stores electric power. The power supply 111 supplies the electric power to each structural element of the inhalation device 1. The power supply 111 may be constituted by, for example, a rechargeable battery such as a lithium ion secondary battery. The power supply 111 may be charged by being connected to an external power supply via a universal serial bus (USB) cable or the like. In addition, the power supply 111 may be charged in a state of not being connected to a power-transmission device by a wireless power transmission technology. Alternatively, only the power supply 111 may be detachable from the inhalation device 1, and may be replaceable with a new power supply 111.

The sensor 112 detects various kinds of information related to the inhalation device 1. As an example, the sensor 112 includes a pressure sensor 112p such as a microphone capacitor, a flow rate sensor 112q for detecting the amount of the aerosol source stored in the liquid storage 123, and a temperature sensor 112t for detecting the temperature of the heater 121. Then, the sensor 112 outputs the detected information to the controller 116. For example, in a case where the pressure sensor 112p detects a numerical value associated with the user's inhalation, the sensor 112 outputs information indicating that the user inhales to the controller 116.

The notifier 113 notifies the user of information. As an example, the notifier 113 is constituted by a light-emitting device such as a light emitting diode (LED). In this case, the notifier 113 emits light in different patterns of light in a case where the state of the power supply 111 is required to be charged, in a case where the power supply 111 is being charged, and in a case where an abnormality occurs in the inhalation device 1. Here, the pattern of light is a concept including a color, timing of turning on/off, and the like. The notifier 113 may be constituted by a display device that displays an image, a sound output device that outputs sound, a vibration device that vibrates, or the like, together with or instead of the light-emitting device.

The memory 114 stores various kinds of information for the operations of the inhalation device 1. The memory 114 may be constituted by, for example, a non-volatile storage medium such as flash memory. An example of the information stored in the memory 114 is information related to an operating system (OS) of the inhalation device 1, such as control details of various structural elements by the controller 116. Another example of the information stored in the memory 114 is information related to the user's inhalation, such as the number of times of inhalation, an inhalation time, and an accumulated inhalation time period.

The communicator 115 is a communication interface for transmitting and receiving information between the inhalation device 1 and another device. The communicator 115 performs communication in conformity with any wired or wireless communication standard. Such a communication standard may be, for example, wireless local area network (LAN), wired LAN, Wi-Fi (registered trademark), Bluetooth (registered trademark), or the like. As an example, the communicator 115 transmits the information related to the user's inhalation to a smartphone in order to cause the smartphone to display the information related to the user's inhalation. As another example, the communicator 115 receives information of a new OS from a server in order to update the information of the OS stored in the memory 114.

The controller 116 functions as an arithmetic processing device and a control device, and controls the overall operations of the inhalation device 1 in accordance with various programs. The controller 116 is implemented by, for example, an electronic circuit such as a central processing unit (CPU) or a microprocessor. The controller 116 may further include a read only memory (ROM) that stores programs to be used, calculation parameters, and the like, and a random access memory (RAM) that temporarily stores parameters and the like that change as appropriate. The inhalation device 1 performs various kinds of processing under the control of the controller 116. The supply of electric power from the power supply 111 to the other structural elements, the charging of the power supply 111, the detection of information by the sensor 112, the notification of information by the notifier 113, the storing and reading of information by the memory 114, and the transmission and reception of information by the communicator 115 are examples of processing controlled by the controller 116. Other processing executed by the inhalation device 1, such as input of information to each structural element and processing based on information output from each structural element, is also controlled by the controller 116.

The operation portion 117 is constituted by a button-type switch, a touch panel, or the like. The operation portion 117 outputs information operated by the user to the controller 116. For example, when a predetermined activation operation is performed on the operation portion 117 in a state in which the power supply unit 110 is powered off, the operation portion 117 outputs an activation command of the power supply unit 110 to the controller 116. Upon receiving the activation command, the controller 116 activates the power supply unit 110. As an example, the predetermined activation operation by the operation portion 117 may be the operation portion 117 being quickly pressed three times in a row.

The DC/DC converter 118 is connected between the heater 121 and the power supply 111 in a state in which the cartridge 120 is attached to the power supply unit 110. The controller 116 is connected between the DC/DC converter 118 and the power supply 111.

The DC/DC converter 118 is a booster circuit capable of boosting an input voltage, and is configured to be capable of supplying a voltage obtained by boosting the input voltage or the input voltage to the heater 121. The DC/DC converter 118 can adjust electric power supplied to the heater 121. As the DC/DC converter 118, for example, a switching regulator that converts an input voltage into a desired output voltage by controlling the on/off time of a switching element while monitoring the output voltage can be used. In a case where a switching regulator is used as the DC/DC converter 118, the input voltage can be output as it is without being boosted by controlling a switching element.

The temperature sensor 112t includes a voltage sensor and a current sensor. The voltage sensor measures and outputs the value of voltage applied to the heater 121. The current sensor measures and outputs the value of current flowing through the heater 121. Each of the output of the voltage sensor and the output of the current sensor is input to the controller 116. The controller 116 acquires the resistance value of the heater 121 based on the output of the voltage sensor and the output of the current sensor, and acquires the temperature of the heater 121 corresponding to the resistance value. The temperature of the heater 121 can be considered to be substantially the same as the temperature of the aerosol source heated by the heater 121.

Note that the temperature sensor 112t does not necessarily include the current sensor if a constant current is supplied to the heater 121 when the resistance value of the heater 121 is acquired. Similarly, the temperature sensor 112t does not necessarily include the voltage sensor if a constant voltage is applied to the heater 121 when the resistance value of the heater 121 is acquired.

In addition, the temperature sensor 112t may be, for example, a thermistor disposed in the vicinity of the heater 121.

### (Cartridge 120)

The liquid storage 123 stores the aerosol source. The aerosol source is heated to be atomized and produces aerosol. Examples of the aerosol source include, for example, polyhydric alcohols such as glycerin and propylene glycol, and liquids such as water. The aerosol source may further include a tobacco raw material or an extract derived from a tobacco raw material, which is heated to release flavor components. The aerosol source may further include nicotine. In a case where the inhalation device 1 is a medical inhaler, such as a nebulizer, the aerosol source may include medicine for a patient to inhale.

The liquid guide 122 guides the aerosol source, which is the liquid stored in the liquid storage 123, from the liquid storage 123 and holds the aerosol source. The liquid guide 122 according to this embodiment is a wick formed by twisting a fiber material such as a glass fiber or a porous material such as a porous ceramic. The liquid guide 122 is in fluid communication with the liquid storage 123. Therefore, the aerosol source stored in the liquid storage 123 spreads over the entire liquid guide 122 by the capillary action.

The heater 121 heats the aerosol source to atomize the aerosol source and generates aerosol. The heater 121 is formed of any material such as metal or polyimide in any shape such as a coil shape, a film shape, or a blade shape. The heater 121 is disposed close to the liquid guide 122. In the examples illustrated in Figs. 2 and 3, the heater 121 is formed of a metal coil and is wound around the liquid guide 122. Therefore, when the heater 121 produces heat, the aerosol source held in the liquid guide 122 is heated and atomized, and aerosol is generated. The heater 121 produces heat when receiving electric power from the power supply 111.

### (Airflow Path 180)

The airflow path 180 is a flow path of air to be inhaled by the user. The airflow path 180 has an air inlet hole 181, which is an inlet of air into the airflow path 180, and an air outlet hole 182, which is an outlet of air from the airflow path 180, at both ends. As the user inhales, air flows into the airflow path 180 through the air inlet hole 181, and air flows out of the airflow path 180 through the air outlet hole 182. As an example, the air inlet hole 181 may be formed around the operation portion 117. The air outlet hole 182 is formed in the mouthpiece 124.

The liquid guide 122 is disposed in the middle of the airflow path 180. The aerosol generated by the heater 121 is mixed with the air that has flowed in through the air inlet hole 181. As the user inhales, the mixture fluid of the aerosol and the air is conveyed to the air outlet hole 182 as indicated by an arrow 190.

### (Case 10)

The case 10 includes a cylindrical power supply unit case 11 that accommodates the power supply unit 110, and a cylindrical cartridge case 12 that accommodates the cartridge 120.

The power supply unit case 11 is provided with the operation portion 117, which is operable by the user, in a state of being exposed from the surface of the power supply unit case 11. In the power supply unit case 11, the air inlet hole 181 for taking in outside air is formed. As an example, the air inlet hole 181 may be formed around the operation portion 117. The pressure sensor 112p is provided in the vicinity of the operation portion 117. The pressure sensor 112p is configured to output the value of a pressure change in the power supply unit 110 caused by the user's inhalation through the mouthpiece 124. For example, the pressure sensor 112p outputs an output value corresponding to the flow rate of air inhaled from the air inlet hole 181 toward the mouthpiece 124, in other words, the pressure that changes according to the user's inhalation.

### (End Cap 20)

The end cap 20 includes a first cylindrical portion 21 and a second cylindrical portion 22. The first cylindrical portion 21 is cylindrical and fitted into an opening of the cartridge case 12 on a side opposite to the power supply unit case 11. The second cylindrical portion 22 is cylindrical and provided outside the cartridge case 12. A part of the first cylindrical portion 21 on the cartridge case 12 side is fitted into the cartridge case 12, and the first cylindrical portion 21 has a flange portion that abuts against an end surface of the cartridge case 12. The diameter of the outer circumference surface of the second cylindrical portion 22 is smaller than the diameter of the outer circumference surface of the first cylindrical portion 21, and the diameter of the inner circumference surface of the second cylindrical portion 22 is the same as the diameter of the inner circumference surface of the first cylindrical portion 21.

### (Mouthpiece 124)

The mouthpiece 124 is a cylindrical member, a part of which on the cartridge case 12 side is fitted into the end cap 20, and has a flange portion that abuts against an end surface of the end cap 20.

The mouthpiece 124 is an example of an inhalation port to be held by the user in their mouth during inhalation. The air outlet hole 182 of the airflow path 180 is formed in the mouthpiece 124. The user inhales with the mouthpiece 124 held in their mouth so as to take into the oral cavity, the mixture fluid of the aerosol and the air conveyed through the airflow path 180.

### (Heating Control of Heater 121 by Controller 116)

The controller 116 is activated when the inhalation device 1 is powered on. For example, the inhalation device 1 is powered on in a case where the operation portion 117 is quickly pressed three times in a row.

Then, in a case where a predetermined condition is satisfied, the controller 116 performs electric power supply to the heater 121 so that the temperature of the aerosol source, which is a liquid, becomes higher than or equal to a first temperature at which aerosol is generated by atomization. As an example, the case where the predetermined condition is satisfied may be a case where the output value of the pressure sensor 112p of the sensor 112 becomes larger than or equal to a predetermined threshold value. As an example, the case where the output value of the pressure sensor 112p becomes larger than or equal to the threshold value may be a case where, for example, the user inhales with the mouthpiece 124 held in their mouth, and where the flow rate and pressure of the air inhaled from the air inlet hole 181 toward the mouthpiece 124 change so that the output value of the pressure sensor 112p exceeds the threshold value. Hereinafter, the user's inhalation with the mouthpiece 124 held in their mouth may be referred to as an "inhaling operation". As an example, the first temperature may be the boiling point of the aerosol source.

As described above, for example, when the user performs the inhaling operation, the controller 116 performs the electric power supply to the heater 121 to heat the heater 121 so that the temperature of the aerosol source becomes higher than or equal to the boiling point. Hereinafter, supplying electric power to the heater 121 to heat the heater 121 so that the temperature of the aerosol source becomes higher than or equal to the boiling point may be referred to as "inhalation heating". The controller 116 starts the inhalation heating upon a predetermined condition being satisfied. In addition, the predetermined condition described above may be referred to as an "inhalation heating condition". As an example, the inhalation heating condition may be a condition that the output value of the pressure sensor 112p becomes larger than or equal to the threshold value.

When performing the inhalation heating, the controller 116 performs control such that, for example, the value of the electric power supplied to the heater 121 becomes an electric power value that is determined in advance as an electric power value when the inhalation heating is performed. As an example, the electric power value determined in advance may be a value obtained by performing an experiment or the like in advance and stored in the memory 114 or the ROM. As another example, the electric power value determined in advance may be determined such that the temperature of the heater 121 in the inhalation heating becomes an inhalation heating target temperature, which will be described later.

The controller 116 may set the target temperature of the heater 121 in the inhalation heating to be higher than or equal to the first temperature, and may control the electric power supply so that the temperature of the heater 121 in the inhalation heating becomes the target temperature. Hereinafter, the target temperature of the heater 121 in the inhalation heating may be referred to as the "inhalation heating target temperature". As an example, the inhalation heating target temperature may be 180 °C .

When performing the inhalation heating, the controller 116 may control, for example, the electric power supplied to the heater 121 via the DC/DC converter 118 so that the temperature of the heater 121 detected by the temperature sensor 112t becomes the inhalation heating target temperature. For example, the controller 116 may control the electric power supplied to the heater 121, based on the deviation between the inhalation heating target temperature stored in the memory 114 and an actual temperature of the heater 121 detected by the temperature sensor 112t (hereinafter, may be referred to as "actual temperature"). The temperature control of the heater 121 can be implemented by, for example, known feedback control. Note that the controller 116 may alternatively control the electric power supplied to the heater 121 based on the deviation between a set temperature (hereinafter, may be referred to as an "inhalation heating set temperature") and the actual temperature. The set temperature is lower (for example, 175 degrees) than the inhalation heating target temperature so that the actual temperature does not exceed the inhalation heating target temperature.

As long as the output value of the pressure sensor 112p is larger than or equal to the threshold value, in other words, as long as the user continues the inhaling operation, the controller 116 determines that the inhalation heating condition is satisfied and performs the inhalation heating. However, when a period during which the output value of the pressure sensor 112p is larger than or equal to the threshold value reaches a predetermined upper limit time (for example, 2.4 seconds), the controller 116 stops the electric power supply to the heater 121 regardless of the output value of the pressure sensor 112p.

On the other hand, in a case where an event in which the inhalation heating condition is estimated to be satisfied is detected before the inhalation heating condition is satisfied, the controller 116 performs the electric power supply to the heater 121 so that the temperature of the aerosol source becomes a temperature higher than or equal to a second temperature and lower than the first temperature. As an example, the case where the event in which the inhalation heating condition is estimated to be satisfied is detected may be a case where a predetermined operation determined in advance (for example, pressing once) is performed on the operation portion 117. Note that the predetermined operation may be performed on an operation portion different from the operation portion 117 on which the predetermined activation operation for turning on the power supply unit 110 is performed. As an example, the second temperature may be 40 degrees.

As described above, for example, if the operation portion 117 is pressed once before the user inhales, the controller 116 heats the heater 121 by supplying the electric power to the heater 121 so that the temperature of the aerosol source becomes a temperature higher than or equal to the second temperature and lower than the first temperature. Hereinafter, heating the heater 121 by supplying the electric power to the heater 121 so that the temperature of the aerosol source becomes a temperature higher than or equal to the second temperature and lower than the first temperature may be referred to as "preliminary heating". The controller 116 starts the preliminary heating upon the event in which the inhalation heating condition is estimated to be satisfied being detected. Hereinafter, the event in which the inhalation heating condition is estimated to be satisfied may be referred to as a "preliminary event".

When performing the preliminary heating, the controller 116 performs control such that, for example, the value of the electric power supplied to the heater 121 becomes an electric power value that is determined in advance as an electric power value when the preliminary heating is performed. As an example, the electric power value determined in advance may be a value obtained by performing an experiment or the like in advance and stored in the memory 114 or the ROM. As another example, the electric power value determined in advance may be determined such that the temperature of the heater 121 in the preliminary heating becomes a preliminary heating target temperature, which will be described later.

The controller 116 may set the target temperature of the heater 121 in the preliminary heating to a temperature higher than or equal to the second temperature and lower than the boiling point of the aerosol source, and may control the electric power supply so that the temperature of the heater 121 in the preliminary heating becomes the target temperature. Hereinafter, the target temperature of the heater 121 in the preliminary heating may be referred to as the "preliminary heating target temperature". As an example, the preliminary heating target temperature may be 50 degrees.

When performing the preliminary heating, the controller 116 may control, for example, the electric power supplied to the heater 121 via the DC/DC converter 118 so that the temperature of the heater 121 detected by the temperature sensor 112t becomes the preliminary heating target temperature. For example, the controller 116 may control the electric power supplied to the heater 121 based on the deviation between the preliminary heating target temperature stored in the memory 114 and the actual temperature of the heater 121 detected by the temperature sensor 112t (actual temperature). The temperature control of the heater 121 can be implemented by, for example, known feedback control. Note that the controller 116 may alternatively control the electric power supplied to the heater 121 based on the deviation between a set temperature (hereinafter, may be referred to as a "preliminary heating set temperature") and the actual temperature. The set temperature is lower (for example, 45 degrees) than the preliminary heating target temperature so that the actual temperature does not exceed the preliminary heating target temperature.

In addition, since the preliminary heating target temperature is lower than the inhalation heating target temperature, the controller 116 sets the value of the electric power at the time of the preliminary heating to be smaller than the value of the electric power at the time of the inhalation heating. For example, the controller 116 sets the duty ratio of a PWM signal to be output to the DC/DC converter 118 at the time of the preliminary heating to be smaller than the duty ratio thereof at the time of the inhalation heating. As examples, the duty ratio at the time of the inhalation heating may be set to 90 %, and the duty ratio at the time of the preliminary heating may be set to 30 %.

Until the actual temperature reaches the preliminary heating set temperature, the controller 116 may fix the duty ratio to 30 %, and after the actual temperature reaches the preliminary heating set temperature, the controller 116 may change the duty ratio, based on the deviation between the actual temperature and the set temperature. Similarly, until the actual temperature reaches the set temperature at the time of inhalation, the controller 116 may fix the duty ratio to 90 %, and after the actual temperature reaches the set temperature at the time of inhalation, the controller 116 may change the duty ratio, based on the deviation between the actual temperature and the set temperature.

While performing the preliminary heating, if the inhalation heating condition is satisfied, the controller 116 performs the inhalation heating.

Therefore, in the inhalation device 1, as a process of making transition to the inhalation heating by the controller 116 controlling the electric power supply to the heater 121 as described above, there are a case of making transition to the inhalation heating after performing the preliminary heating and a case of making transition to the inhalation heating without performing the preliminary heating. In the following description, the inhalation heating in a case of making transition to the inhalation heating after performing the preliminary heating may be referred to as "first inhalation heating", and the inhalation heating in a case of making transition to the inhalation heating without performing the preliminary heating may be referred to as "second inhalation heating".

On the other hand, while performing the preliminary heating, if a predetermined condition for ending the preliminary heating (hereinafter, may be referred to as "preliminary heating end condition") is satisfied before the inhalation heating condition is satisfied, the controller 116 stops the preliminary heating. This is to suppress wasteful electric power consumption associated with the preliminary heating. As an example, the preliminary heating end condition may be a condition that a predetermined time (for example, 10 seconds) has elapsed after the start of the preliminary heating.

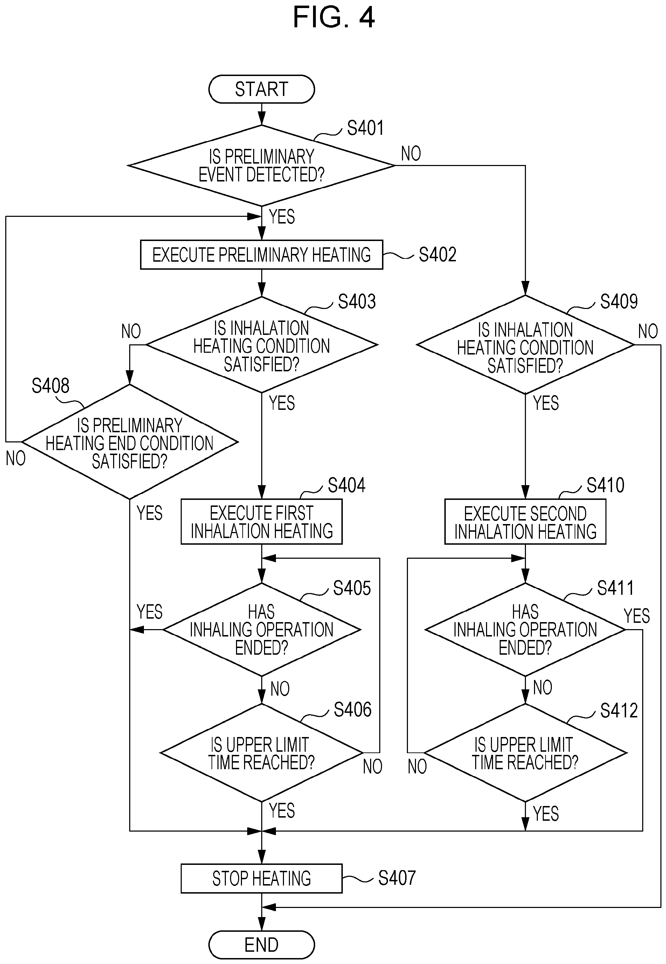

Fig. 4 is a flowchart illustrating an example of a procedure of a heating process performed by the controller 116.

The controller 116 repeatedly executes this process, for example, at a control cycle determined in advance (for example, every 1 millisecond).

The controller 116 determines whether the preliminary event is detected (S401). If the preliminary event is detected (YES in S401), the controller 116 performs the preliminary heating (S402). Subsequently, the controller 116 determines whether the inhalation heating condition is satisfied (S403). If the inhalation heating condition is satisfied (YES in S403), the controller 116 performs the first inhalation heating (S404). Subsequently, it is determined whether the inhaling operation has ended (S405). If it is determined that the inhaling operation has not ended (NO in S405), the controller 116 determines whether an upper limit time is reached (S406). If the upper limit time is not reached (NO in S406), the controller 116 performs the process in and after S405. If the upper limit time is reached (YES in S406), or if the inhaling operation has ended (YES in S405), the controller 116 stops the electric power supply from the power supply 111 to the heater 121 to stop heating (S407).

On the other hand, if it is determined in S403 that the inhaling operation is not performed by the user (NO in S403), the controller 116 determines whether the preliminary heating end condition is satisfied (S408). If the preliminary heating end condition is not satisfied (NO in S408), the controller 116 performs the process in and after S402. On the other hand, if the preliminary heating end condition is satisfied (YES in S408), the controller 116 stops the electric power supply from the power supply 111 to the heater 121 to stop heating (S407).

On the other hand, if it is determined in S401 that the preliminary event is not detected (NO in S401), the controller 116 determines whether the inhalation heating condition is satisfied (S409). If the inhalation heating condition is not satisfied (NO in S409), the controller 116 ends the process. If the inhalation heating condition is satisfied (YES in S409), the controller 116 performs the second inhalation heating (S410). Subsequently, it is determined whether the inhaling operation has ended (S411). If it is determined that the inhaling operation has not ended (NO in S411), the controller 116 determines whether the upper limit time is reached (S412). If the upper limit time is not reached (NO in S412), the controller 116 performs the process in and after S411. If the upper limit time is reached (YES in S412), or if the inhaling operation has ended (YES in S411), the controller 116 stops the electric power supply from the power supply 111 to the heater 121 to stop heating (S407).

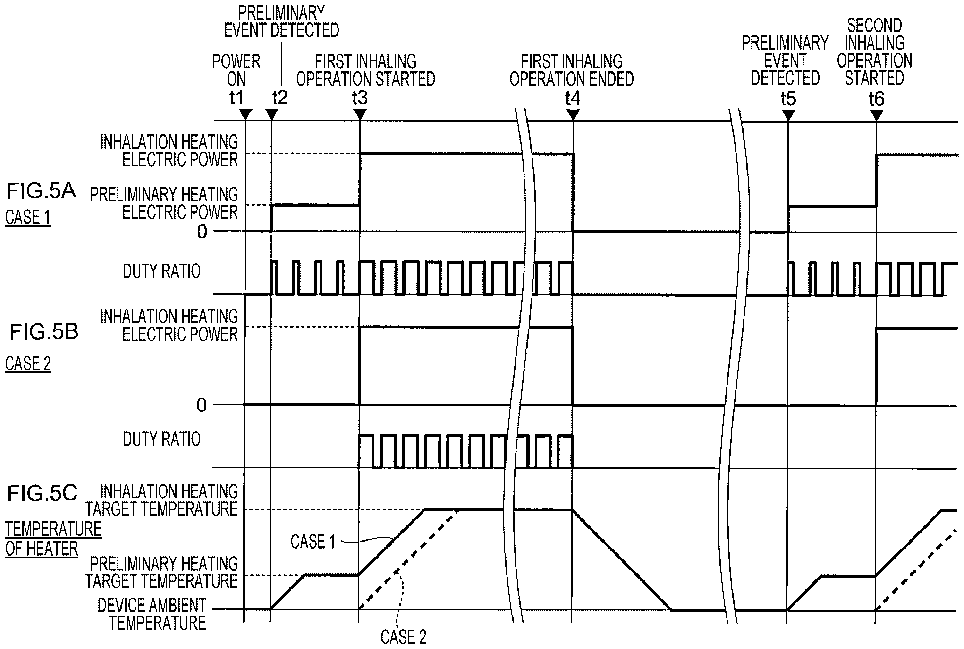

Figs. 5A to 5C are timing charts for describing operations of the inhalation device 1.

Fig. 5A is a timing chart of a case where the first inhalation heating is performed, and Fig. 5B is a timing chart of a case where the second inhalation heating is performed.

More specifically, Fig. 5A illustrates an operation in a case where an operation for powering on the inhalation device 1 is performed at time t1, the preliminary event is detected at subsequent time t2, and it is detected that a first inhaling operation is performed (it is detected that the inhalation heating condition is satisfied) at subsequent time t3. In addition, Fig. 5A illustrates an operation in a case where it is detected that the first inhaling operation is not performed at time t4, the preliminary event is detected at subsequent time t5, and it is detected that a second inhaling operation is performed at subsequent time t6.

Fig. 5B illustrates an operation in a case where an operation for powering on the inhalation device 1 is performed at time t1, and it is detected that a first inhaling operation is performed (it is detected that the inhalation heating condition is satisfied) at subsequent time t3. In addition, Fig. 5B illustrates an operation in a case where it is detected that the first inhaling operation is not performed at time t4, and it is detected that a second inhaling operation is performed at subsequent time t6.

Fig. 5C illustrates changes in the temperature of the heater 121 in a case where the inhalation device 1 operates as illustrated in Fig. 5A (hereinafter, may be referred to as "case 1") and in a case where the inhalation device 1 operates as illustrated in Fig. 5B (hereinafter, may be referred to as "case 2"). The solid line indicates changes in the temperature in the case 1, and the broken line indicates changes in the temperature in the case 2.

As illustrated in Fig. 5C, in the case 1, since the preliminary heating is performed before the inhalation heating is performed, the inhalation heating target temperature is likely to be reached earlier than in the case 2. Therefore, in the case 1, the temperature of the aerosol source is likely to reach the temperature at which aerosol is generated by atomization earlier than in the case 2.

Therefore, in the inhalation device 1, the amount of aerosol generated at the initial stage of inhalation by the user is larger in a case of performing the first inhalation heating (case 1 in Figs. 5A to 5C) than in a case of performing the second inhalation heating (case 2 in Figs. 5A to 5C). This is due to the following reasons.

The liquid guide 122 guides and holds the aerosol source, which is a liquid stored in the liquid storage 123, by the capillary action, and the heater 121 is disposed close to the liquid guide 122 and produces heat to atomize the aerosol source and generates aerosol. Therefore, as the amount of the electric power supplied to the heater 121 increases, the amount of generated aerosol increases.

In the second inhalation heating, electric power is supplied to the heater 121 after the user performs the inhaling operation. Thus, most of the electric power supplied at the initial stage of inhalation is consumed to increase the temperature of the liquid, which is the aerosol source, and the amount of electric power consumed to vaporize the liquid decreases. As a result, the amount of aerosol generated at the initial stage of inhalation is reduced.

On the other hand, in the first inhalation heating to which transition is made after the preliminary heating, electric power is supplied to the heater 121 before the user performs the inhaling operation, and the temperature of the liquid, which is the aerosol source, is increased. Therefore, in the first inhalation heating, the amount of electric power consumed to increase the temperature of the liquid is smaller than that in the second inhalation heating, and the amount of electric power consumed to vaporize the liquid is larger than that in the second inhalation heating, from the electric power supplied at the initial stage of inhalation. As a result, the amount of aerosol generated at the initial stage of inhalation is larger in the first inhalation heating than in the second inhalation heating.

As described above, the inhalation device 1 includes the liquid storage 123, the heater 121, the power supply 111, and the controller 116. The liquid storage 123 stores the liquid, which is the aerosol source for generating aerosol by being heated. The heater 121 heats the liquid. The power supply 111 accumulates electric power. The controller 116 controls the electric power supply from the power supply 111 to the heater 121. In addition, if the inhalation heating condition as an example of a predetermined condition determined in advance is satisfied, the controller 116 controls the electric power supply to perform the inhalation heating as an example of first heating in which the temperature of the liquid, which is the aerosol source, becomes higher than or equal to the first temperature (for example, the boiling point) at which the liquid is vaporized. On the other hand, if the event in which the inhalation heating condition is estimated to be satisfied is detected before the inhalation heating condition is satisfied, the controller 116 controls the electric power supply to perform the preliminary heating as an example of second heating in which the temperature of the liquid, which is the aerosol source, becomes a temperature higher than or equal to the second temperature (for example, 40 degrees) and lower than the first temperature (for example, the boiling point).

That is, the inhalation device 1 performs the preliminary heating if the event in which the inhalation heating condition is estimated to be satisfied is detected before the inhalation heating condition is satisfied, and then performs the inhalation heating if the inhalation heating condition is satisfied. According to the inhalation device 1 configured as described above, since the inhalation heating is performed after the preliminary heating is performed, the amount of aerosol at the initial stage of inhalation is larger than that in a case where the inhalation heating is performed without performing the preliminary heating.

Although the second temperature is 40 degrees as an example, the second temperature is not particularly limited to 40 degrees. Since the purpose of the preliminary heating is to increase the temperature of the liquid, which is the aerosol source, in advance before the inhalation heating is performed, the second temperature may be higher than the temperature of a place where the inhalation device 1 is used. For example, in a case where the region where the inhalation device 1 is used is Japan, the second temperature may be higher than the atmospheric temperature in Japan. Since the air temperature changes according to the season, the second temperature may be changed according to the season. In addition, although the preliminary heating target temperature is 50 degrees as an example, the preliminary heating target temperature is not particularly limited to 50 degrees. The preliminary heating target temperature may be changed in the same manner as the change of the second temperature, for example, by setting the preliminary heating target temperature to the second temperature + 10 degrees. Similarly, in a case where the value of the electric power supplied to the heater 121 at the time of the preliminary heating is set to an electric power value determined in advance, the electric power value determined in advance may be changed in the same manner as the change in the second temperature. That is, the electric power value determined in advance and the preliminary heating target temperature may be changed according to the region and the season in which the inhalation device 1 is used.

In the inhalation device 1, since the preliminary heating is performed if the event in which the inhalation heating condition is estimated to be satisfied is detected before the inhalation heating condition is satisfied, for example, it is possible to suppress wasteful electric power consumption associated with the preliminary heating as compared with a case where the preliminary heating is started when the inhalation device 1 is powered on. That is, for example, even if the inhalation device 1 is powered on, the user does not always perform the inhaling operation immediately. If the inhaling operation is not performed after the preliminary heating is started when the inhalation device 1 is powered on, the electric power required to perform the preliminary heating is wasted. In contrast, according to the inhalation device 1, after the inhalation device 1 is powered on, if the event in which the inhalation heating condition is estimated to be satisfied is detected, for example, in a case where the predetermined operation (for example, pressing once) is performed on the operation portion 117, the preliminary heating is started. In addition, the event in which the inhalation heating condition is estimated to be satisfied leads to the user's inhaling operation with higher accuracy than other matters (for example, the inhalation device 1 being powered on), since the inhalation heating is performed with high accuracy after the preliminary heating is performed, and thus, the electric power required to perform the preliminary heating is unlikely to be wasted.

In addition, according to the inhalation device 1, for example, as compared with a configuration in which the preliminary heating is performed throughout a period between an n-th inhaling operation and an (n + 1)-th inhaling operation performed subsequently to the n-th inhaling operation, it is possible to suppress wasteful electric power consumption associated with the preliminary heating. That is, since the time interval between the n-th inhaling operation and the (n + 1)-th inhaling operation is about 10 to 20 seconds, for example, after the event in which the inhalation heating condition is estimated to be satisfied is detected, if the time interval until the inhaling operation is performed is about 3 seconds, the period for the preliminary heating can be shortened by 7 to 17 seconds. Then, if the preliminary heating target temperature is reached within 3 seconds after the start of the preliminary heating, it is possible to suppress electric power necessary to perform the preliminary heating for the period for which the preliminary heating can be shortened.

In addition, in the inhalation device 1, the controller 116 sets the value of the electric power at the time of the preliminary heating to be smaller than the value of the electric power at the time of the inhalation heating. For example, the controller 116 sets the duty ratio at the time of the inhalation heating to 90 % and the duty ratio at the time of the preliminary heating to 30 %. Accordingly, even if the inhaling operation is not performed after the preliminary heating is performed, it is possible to suppress wasteful electric power consumption associated with the preliminary heating.

In addition, the controller 116 performs control such that the temperature of the heater 121 does not exceed the target temperature. Accordingly, since it is possible to suppress the temperature of the heater 121 from being increased more than necessary, it is possible to suppress wasteful electric power consumption associated with the preliminary heating.

### (Modifications of Detection of Event in Which Inhalation Heating Condition Is Estimated To Be Satisfied)

Hereinafter, modifications of the detection of the event in which the inhalation heating condition is estimated to be satisfied, in other words, the detection of the event in which the inhaling operation is estimated to be performed, will be described. Hereinafter, the event in which the inhalation heating condition is estimated to be satisfied may be referred to as a "preliminary event".

Here, by performing the preliminary heating before the inhaling operation is performed, a high atomization amount can be inhaled from the initial stage of inhalation. However, after the preliminary heating is performed, if the inhaling operation is not performed, electric power for the preliminary heating is wasted. In addition, when the time until the preliminary heating target temperature is reached after the preliminary heating is started is referred to as a "minimum heating time", if the preliminary heating is started the minimum heating time before the inhaling operation is performed, it is possible to suppress electric power consumption for maintaining the temperature at the preliminary heating target temperature after the preliminary heating target temperature is reached. Although depending on the specification of the heater 121 and the preliminary heating target temperature, as an example, the minimum heating time may be 2 seconds or less. In a case where the minimum heating time is 2 seconds, if the preliminary heating is started 2 seconds before the inhaling operation is performed, the temperature can be sufficiently set to the preliminary heating target temperature when the inhaling operation is performed.

From the above, it is desirable to start the preliminary heating the minimum heating time before the inhaling operation is performed with high accuracy.

As the preliminary event, in addition to the above-described predetermined operation (for example, pressing once) on the operation portion 117, the following are considered.
(1) The inhalation device 1 has been moved to the mouth. This is because the user moves the inhalation device 1 to the mouth before performing the inhaling operation. In particular, it is considered that the inhalation device 1 is moved to the mouth for the first inhaling operation.
(2) The inhalation device 1 is in the vicinity of the mouth. This is because the inhalation device 1 is in the vicinity of the mouth when the user performs the inhaling operation. In particular, before the second and subsequent inhaling operations, it is considered that the inhalation device 1 is stayed in the vicinity of the mouth continuously after the previous inhaling operation.
(3) The inhalation device 1 has touched the lips. This is because the user holds the mouthpiece 124 in their mouth when performing the inhaling operation.

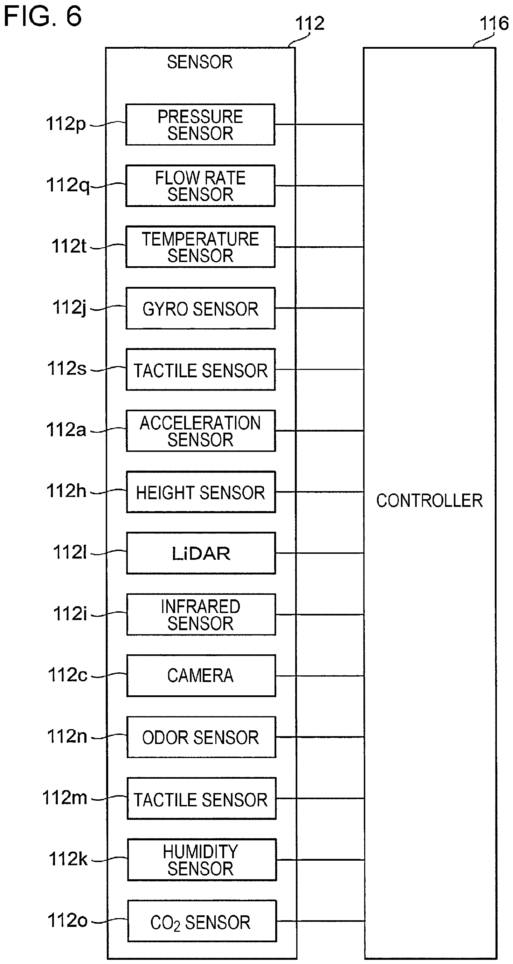

Fig. 6 is a diagram illustrating an example of a schematic configuration of the sensor 112 and the controller 116 according to the modifications.

In a case of the above (1), as an example, the controller 116 may detect the preliminary event as follows.

It is considered that the user holds and lifts the inhalation device 1 placed on a desk or a table, for example, before performing the inhaling operation. Thus, as an example, the sensor 112 may include a gyro sensor 112j, and the controller 116 may detect the preliminary event if the output value of the gyro sensor 112j indicates that the orientation of the inhalation device 1 has been changed from horizontal to vertical. As an example, the gyro sensor 112j may be provided in the power supply unit case 11. When the inhalation device 1 is placed on a desk or a table, the inhalation device 1 is in the horizontal orientation in a state in which the power supply 111 and the mouthpiece 124 are at the same height. On the other hand, when the user is performing the inhaling operation, as illustrated in Fig. 1, the inhalation device 1 is in the vertical orientation in a state in which the mouthpiece 124 is positioned above the power supply unit 110, in other words, in a state in which the height of the mouthpiece 124 is higher than the height of the power supply 111. Therefore, as an example, the controller 116 may detect the preliminary event if the output value of the gyro sensor 112j changes from a value indicating that the power supply 111 and the mouthpiece 124 are at the same height to a value indicating that the height of the mouthpiece 124 is higher than the height of the power supply 111. Note that the state in which the power supply 111 and the mouthpiece 124 are at the same height is not limited to a case where the power supply 111 and the mouthpiece 124 are exactly at the same height, and may be, for example, a case where the height difference between the power supply 111 and the mouthpiece 124 is less than or equal to 1 cm. This is because, as long as the height difference between the power supply 111 and the mouthpiece 124 is less than or equal to 1 cm, it can be considered that the inhalation device 1 is in the horizontal orientation.

In addition, since the user touches the inhalation device 1 with their hand before performing the inhaling operation, the sensor 112 may include a tactile sensor 112s, and the controller 116 may detect the preliminary event if the output value of the tactile sensor 112s indicates that the hand touches the inhalation device 1. As an example, the tactile sensor 112s may be attached to the power supply unit case 11 in a state of being exposed from the surface of the power supply unit case 11 that accommodates the power supply unit 110.

In addition, it is considered that the user moves the inhalation device 1 from the vicinity of the waist to the mouth, for example, before performing the inhaling operation. Thus, the sensor 112 may include an acceleration sensor 112a, and the controller 116 may detect the preliminary event if the output value of the acceleration sensor 112a becomes larger than or equal to a threshold value determined in advance. If the inhalation device 1 is moved from down to up, a downward inertial force acts, and the acceleration sensor 112a indicates a positive acceleration. If the inhalation device 1 is moved from up to down, an upward inertial force acts, and the acceleration sensor 112a indicates a negative acceleration. Therefore, if the output value of the acceleration sensor 112a becomes larger than or equal to the threshold value determined in advance, it can be considered that the user has been moved the inhalation device 1 from the vicinity of the waist to the mouth in order to perform the inhaling operation. As an example, the acceleration sensor 112a may be provided in the power supply unit case 11.

If the inhalation device 1 is moved from the vicinity of the waist to the mouth, it is considered that the height of the inhalation device 1 changes by the height between the vicinity of the waist and the mouth. Thus, the sensor 112 may include a height sensor 112h, and the controller 116 may detect the preliminary event if the amount of change in the output value of the height sensor 112h becomes larger than or equal to a threshold value determined in advance. As an example, the height sensor 112h may be provided in the power supply unit case 11. Instead of using the output value of the height sensor 112h, the controller 116 may estimate that the inhalation device 1 has been moved from the vicinity of the waist to the mouth if the amount of change in the output value of the pressure sensor 112p becomes larger than or equal to a threshold value determined in advance, and detect the preliminary event.

In addition, if the user moves the inhalation device 1 to the mouth before performing the inhaling operation, the distance between the inhalation device 1 and the mouth decreases. Thus, the inhalation device 1 may include a LiDAR (Light Detection and Ranging) 1121 that measures the distance between the inhalation device 1 and the mouth, and the controller 116 may detect the preliminary event if the output value of the LiDAR 1121 indicates that the distance between the inhalation device 1 and the mouth is smaller than or equal to a threshold value determined in advance. If the user moves the inhalation device 1 to the mouth in order to perform the inhaling operation, the user usually moves the inhalation device 1 upward to the mouth from a position below the mouth. Therefore, as an example, the LiDAR 1121 may measure the distance to the lower lip, the controller 116 may detect the preliminary event if the distance measured by the LiDAR 1121 becomes smaller than or equal to a threshold value determined in advance. Alternatively, the LiDAR 1121 may measure the distance to the nose, and the controller 116 may estimate, by using the distance measured by the LiDAR 1121 and the distance between the nose and a contact portion of the upper lip and the lower lip, which is stored in advance in the memory 114 or the ROM, the distance between the contact portion of the upper lip and the lower lip and the inhalation device 1, and detect the preliminary event if the estimated distance becomes smaller than or equal to a threshold value determined in advance. As an example, the LiDAR 1121 may be attached to the end cap 20. Alternatively, the LiDAR 1121 may be attached to the mouthpiece 124.

In addition, by utilizing the fact that an infrared sensor 112i can measure the body temperature of the user if the inhalation device 1 is moved to the user's mouth, the inhalation device 1 may include the infrared sensor 112i, and the controller 116 may detect the preliminary event if the output value of the infrared sensor 112i becomes larger than or equal to a threshold value determined in advance. As an example, the infrared sensor 112i may be attached to the end cap 20. Alternatively, the infrared sensor 112i may be attached to the mouthpiece 124.

In addition, the inhalation device 1 may include a camera 112c, and the controller 116 may detect the preliminary event if an image captured by the camera 112c indicates that the inhalation device 1 has approached the user's mouth. The image captured by the camera 112c may be a still image or a moving image. In a case of a still image, the camera 112c may capture images, for example, every 1 millisecond. As an example, the camera 112c may be attached to the end cap 20. Alternatively, the camera 112c may be attached to the mouthpiece 124.

In addition, in a case of the above (2), as an example, the controller 116 may detect the preliminary event as follows.

By utilizing the fact that an odor sensor 112n can measure a volatile sulfide compound generated in the user's mouth if the inhalation device 1 is in the vicinity of the user's mouth, the inhalation device 1 may include the odor sensor 112n, and the controller 116 may detect the preliminary event if the output value of the odor sensor 112n is larger than or equal to a threshold value determined in advance. Alternatively, a sensor capable of measuring the flavor components contained in aerosol that can be inhaled by using the inhalation device 1 may be used as the odor sensor 112n, and the controller 116 may detect the preliminary event if the output value of the odor sensor 112n is larger than or equal to a threshold value determined in advance.

Since the user's breath is very humid, if a humidity sensor 112k is in the vicinity of the user's mouth, the output value of the humidity sensor 112k becomes larger than or equal to a predetermined threshold value. Considering this fact, the sensor 112 may include the humidity sensor 112k, and the controller 116 may detect the preliminary event if the output value of the humidity sensor 112k becomes larger than or equal to the threshold value determined in advance.

Since the user's breath has a higher CO₂ concentration than the outside air, if a CO₂ sensor 112o is in the vicinity of the user's mouth, the output value of the CO₂ sensor 112o becomes larger than or equal to a threshold value determined in advance. Considering this fact, the sensor 112 may include the CO₂ sensor 112o, and the controller 116 may detect the preliminary event if the output value of the CO₂ sensor 112o becomes larger than or equal to the threshold value determined in advance.

As an example, the odor sensor 112n, the humidity sensor 112k, and the CO₂ sensor 112o may be attached to the end cap 20. Alternatively, the odor sensor 112n, the humidity sensor 112k, and the CO₂ sensor 112o may be attached to the mouthpiece 124.

In a case of the above (2), as in a case of the above (1), the controller 116 may determine that the inhalation device 1 is in the vicinity of the mouth if the output value of the infrared sensor 112i is larger than or equal to the threshold value determined in advance, and detect the preliminary event. In addition, the controller 116 may detect the preliminary event if the output value of the LiDAR 1121 indicates that the distance between the inhalation device 1 and the mouth is smaller than or equal to the threshold value determined in advance. Furthermore, the controller 116 may detect the preliminary event if the camera 112c captures an image indicating that the inhalation device 1 is in the vicinity of the user's mouth.

In addition, in a case of the above (3), as an example, a tactile sensor 112m may be attached in a state of being exposed from the surface of the mouthpiece 124, and the controller 116 may detect the preliminary event if the output value of the tactile sensor 112m indicates that the mouth is in contact with the mouthpiece 124.

Note that the inhalation device 1 may include at least two or more of the gyro sensor 112j, the tactile sensor 112s, the acceleration sensor 112a, the height sensor 112h, the LiDAR 1121, the infrared sensor 112i, the camera 112c, the odor sensor 112n, the tactile sensor 112m, the humidity sensor 112k, and the CO₂ sensor 112o, which are described above, and the controller 116 may detect the preliminary event based on the output values from the two or more sensors or the like. For example, in the first inhaling operation, the controller 116 may detect the preliminary event if the output value of the gyro sensor 112j indicates that the orientation of the inhalation device 1 is vertical and the output value of the acceleration sensor 112a indicates that the inhalation device 1 has moved from down to up. In the second and subsequent inhaling operations, the controller 116 may detect the preliminary event if the output value of the gyro sensor 112j indicates that the orientation of the inhalation device 1 is vertical and the output value of the infrared sensor 112i is larger than or equal to the threshold value determined in advance. Accordingly, it is possible to detect the preliminary event with higher accuracy.

The inhalation device 1 may include at least three or more of the gyro sensor 112j, the tactile sensor 112s, the acceleration sensor 112a, the height sensor 112h, the LiDAR 1121, the infrared sensor 112i, the camera 112c, the odor sensor 112n, the tactile sensor 112m, the humidity sensor 112k, and the CO₂ sensor 112o, which are described above, and the controller 116 may detect the preliminary event based on the output values from the three or more sensors or the like. For example, in the first inhaling operation, the controller 116 may detect the preliminary event if the output value of the gyro sensor 112j indicates that the orientation of the inhalation device 1 is vertical, the output value of the acceleration sensor 112a indicates that the inhalation device 1 has moved from down to up, and the output value of the infrared sensor 112i is larger than or equal to the threshold value determined in advance. In the second and subsequent inhaling operations, the controller 116 may detect the preliminary event if the output value of the gyro sensor 112j indicates that the orientation of the inhalation device 1 is vertical, the output value of the infrared sensor 112i is larger than or equal to the threshold value determined in advance, and the output value of the odor sensor 112n is larger than or equal to the threshold value determined in advance. Accordingly, it is possible to detect that the preliminary event with higher accuracy.

In addition, the inhalation device 1 may learn the time interval between successive inhaling operations, and the controller 116 may determine the preliminary event if the minimum heating time before the time at which an (n + 1)-th inhaling operation is expected to be started is reached after an n-th inhaling operation. For example, the controller 116 calculates an average value of time intervals between successive inhaling operations, and stores the average value in the memory 114 as an average time interval. The controller 116 may determine the preliminary event if an (average time interval - minimum heating time) has elapsed after the n-th inhaling operation. For example, in a case where the average time interval is 15 seconds and the minimum heating time is 2 seconds, the controller 116 may detect that the preliminary event if 13 seconds have elapsed after the n-th inhaling operation.

### (Regarding End of Preliminary Heating)

As described above, in the inhalation device 1, the controller 116 stops the preliminary heating if the preliminary heating end condition is satisfied after the preliminary heating is performed. For example, the controller 116 stops the preliminary heating when a time (for example, 10 seconds) determined in advance elapses after the start of the preliminary heating. Therefore, compared to a configuration in which the preliminary heating is continued until the inhaling operation is performed after the start of the preliminary heating, it is possible to shorten a period in which the preliminary heating is performed, and thus, it is possible to suppress electric power consumption for the preliminary heating.

Note that the preliminary heating end condition may be the following condition in addition to the above-described condition that the time (for example, 10 seconds) determined in advance elapses after the start of the preliminary heating.

As an example, the controller 116 may set, as the preliminary heating end condition, a condition that the output value of the gyro sensor 112j indicates that the orientation of the inhalation device 1 has been changed from vertical to horizontal. In other words, as an example, the controller 116 may set, as the preliminary heating end condition, a condition that the output value of the gyro sensor 112j changes from a value indicating that the height of the mouthpiece 124 is higher than the height of the power supply 111 to a value indicating that the power supply 111 and the mouthpiece 124 are at the same height. This is because, if the inhalation device 1 is placed on, for example, a desk or a table, the inhaling operation is unlikely to be performed within the minimum heating time.

In addition, the controller 116 may set, as the preliminary heating end condition, a condition that the output value of the tactile sensor 112s does not indicate that a hand is in contact with the inhalation device 1. This is because, if the user releases the hand from the inhalation device 1, the inhaling operation is unlikely to be performed within the minimum heating time.

In addition, the controller 116 may set, as the preliminary heating end condition, a condition that the output value of the acceleration sensor 112a, which indicates a negative acceleration if the inhalation device 1 is moved from up to down, becomes smaller than or equal to a negative threshold value determined in advance. This is because, if the user moves the inhalation device 1 from the mouth to the vicinity of the waist, for example, the inhaling operation is unlikely to be performed within the minimum heating time.

In addition, considering that the amount of change in the height becomes a negative value if the inhalation device 1 is moved from up to down, the controller 116 may set, as the preliminary heating end condition, a condition that the amount of change in the output value of the height sensor 112h becomes smaller than or equal to a negative threshold value determined in advance. This is because, if the user moves the inhalation device 1 from the mouth to the vicinity of the waist, for example, the inhaling operation is unlikely to be performed within the minimum heating time. Instead of using the output value of the height sensor 112h, the controller 116 may estimate that the inhalation device 1 has been moved from the mouth to the vicinity of the waist if the amount of change in the output value of the pressure sensor 112p becomes smaller than or equal to a negative threshold value determined in advance, and determine that the preliminary heating end condition is satisfied.

In addition, if the distance between the inhalation device 1 and the mouth is large, the inhaling operation is unlikely to be performed within the minimum heating time, and thus, the controller 116 may set the following items as the preliminary heating end condition. In other words, the preliminary heating end condition may be satisfied if the distance to the user's mouth exceeds a threshold value determined in advance. For example, the controller 116 may set, as the preliminary heating end condition, a condition that the output value of the LiDAR 1121 indicates that the distance between the inhalation device 1 and the mouth exceeds the threshold value determined in advance. In addition, the controller 116 may set, as the preliminary heating end condition, a condition that the output value of the infrared sensor 112i becomes smaller than a threshold value determined in advance. Furthermore, the controller 116 may set, as the preliminary heating end condition, a condition that the camera 112c captures an image indicating that the inhalation device 1 is not near the user's mouth. Furthermore, the controller 116 may set, as the preliminary heating end condition, a condition that the output value of the odor sensor 112n is smaller than a threshold value determined in advance. Furthermore, the controller 116 may set, as the preliminary heating end condition, a condition that the output value of the humidity sensor 112k is smaller than a threshold value determined in advance. Furthermore, the controller 116 may set, as the preliminary heating end condition, a condition that the output value of the CO₂ sensor 112o is smaller than a threshold value determined in advance.

Note that the inhalation device 1 may include at least two or more of the gyro sensor 112j, the tactile sensor 112s, the acceleration sensor 112a, the height sensor 112h, the LiDAR 1121, the infrared sensor 112i, the camera 112c, the odor sensor 112n, the tactile sensor 112m, the humidity sensor 112k, and the CO₂ sensor 112o, which are described above, and the controller 116 may determine whether the preliminary heating end condition is satisfied based on the output values from the two or more sensors.

For example, the controller 116 may determine that the preliminary heating end condition is satisfied if the output value of the acceleration sensor 112a indicates that the inhalation device 1 has moved from up to down and the output value of the gyro sensor 112j indicates that the orientation of the inhalation device 1 is horizontal. In addition, the controller 116 may determine that the preliminary heating end condition is satisfied if the output value of the acceleration sensor 112a indicates that the inhalation device 1 has moved from up to down and the output value of the infrared sensor 112i becomes smaller than a threshold value determined in advance. Accordingly, it is possible to more accurately determine that the inhaling operation is not performed within the minimum heating time.

By setting the above-described condition as the preliminary heating end condition, the controller 116 can stop the preliminary heating by determining with high accuracy that the inhaling operation is unlikely to be performed within the minimum heating time. Accordingly, it is possible to suppress wasteful electric power consumption associated with the preliminary heating.

As long as the controller 116 stops the preliminary heating if the preliminary heating end condition is satisfied after the preliminary heating is performed, the timing of starting the preliminary heating is not limited to a case where the event in which the inhalation heating condition is expected to be satisfied is detected. For example, the controller 116 may start the preliminary heating if the inhalation device 1 is powered on and activated, and then may stop the preliminary heating if the preliminary heating end condition is satisfied. In addition, the controller 116 may change the inhalation heating to the preliminary heating at the timing when an n-th inhaling operation ends, and then may stop the preliminary heating if the preliminary heating end condition is satisfied.

### <Second Embodiment>

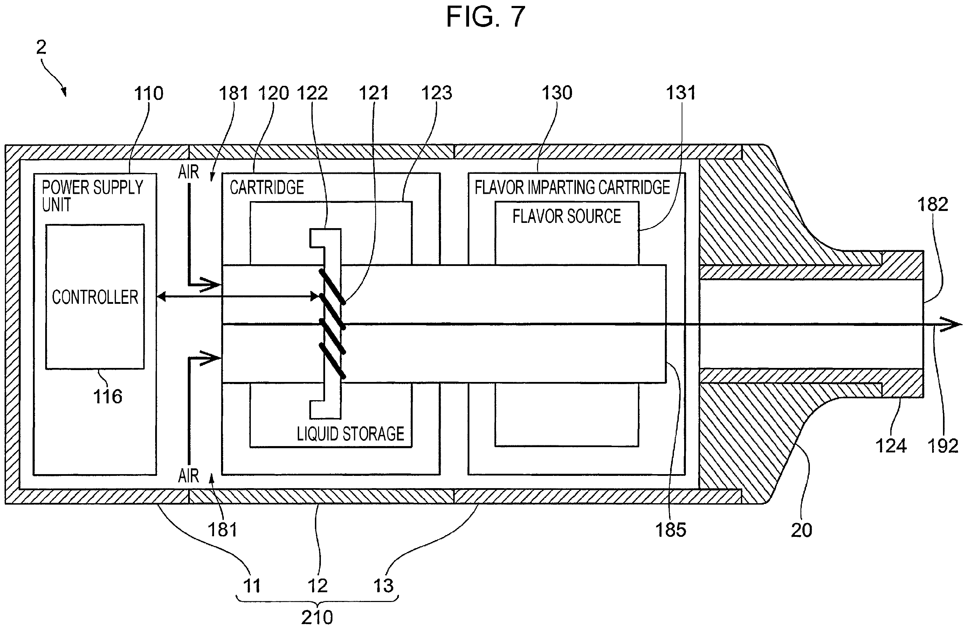

Fig. 7 is a diagram schematically illustrating an example of a schematic configuration of an inhalation device 2 according to a second embodiment.

The inhalation device 2 according to the second embodiment is different from the inhalation device 1 according to the first embodiment in that a flavor imparting cartridge 130 is provided. In addition, the inhalation device 2 is different from the inhalation device 1 in that a case 210 is provided instead of the case 10. Hereinafter, differences from the first embodiment will be described. In the first embodiment and the second embodiment, the same items are denoted by the same reference numerals, and detailed description thereof will be omitted.

The flavor imparting cartridge 130 includes a flavor source 131.

The flavor source 131 is a structural element for imparting a flavor component to aerosol. The flavor source 131 may be derived from tobacco, such as shredded tobacco or a processed product of a tobacco raw material formed into a granular, sheet-like, or powdery form. The flavor source 131 may not be derived from tobacco and may be made from a plant other than tobacco (such as mint and herb). As an example, the flavor source 131 may include a flavor component such as menthol. Note that the flavor source 131 may be disposed inside a container such as a capsule.

In the middle of an airflow path 185, in addition to the liquid guide 122, the flavor source 131 is disposed downstream of (close to the air outlet hole 182) of the liquid guide 122. The aerosol generated by the heater 121 is mixed with air introduced through the air inlet hole 181. Subsequently, as the user inhales, the mixture fluid of the aerosol and the air is conveyed through the flavor source 131 to the air outlet hole 182 as indicated by an arrow 192. Then, when the mixture fluid of the aerosol and the air passes through the flavor source 131, the flavor component contained in the flavor source 131 is imparted to the aerosol.

The case 210 includes, in addition to the power supply unit case 11 and the cartridge case 12, a cylindrical flavor imparting cartridge case 13 that accommodates the flavor imparting cartridge 130. The flavor imparting cartridge 130 and the cartridge 120 are configured to be attachable to and detachable from each other. The end cap 20 is attached to an opening of the flavor imparting cartridge case 13 on a side opposite to the cartridge case 12. The user inhales in a state in which the cartridge 120, the flavor imparting cartridge 130, and the power supply unit 110 are attached to each other, the end cap 20 is attached to the flavor imparting cartridge case 13, and the mouthpiece 124 is attached to the end cap 20.

Also in the inhalation device 2 according to the second embodiment configured as described above, the controller 116 performs the preliminary heating by the same method as that described in the first embodiment, and thus, it is possible to increase the amount of aerosol at the initial stage of inhalation and also to suppress wasteful electric power consumption associated with the preliminary heating.

### <Third Embodiment>

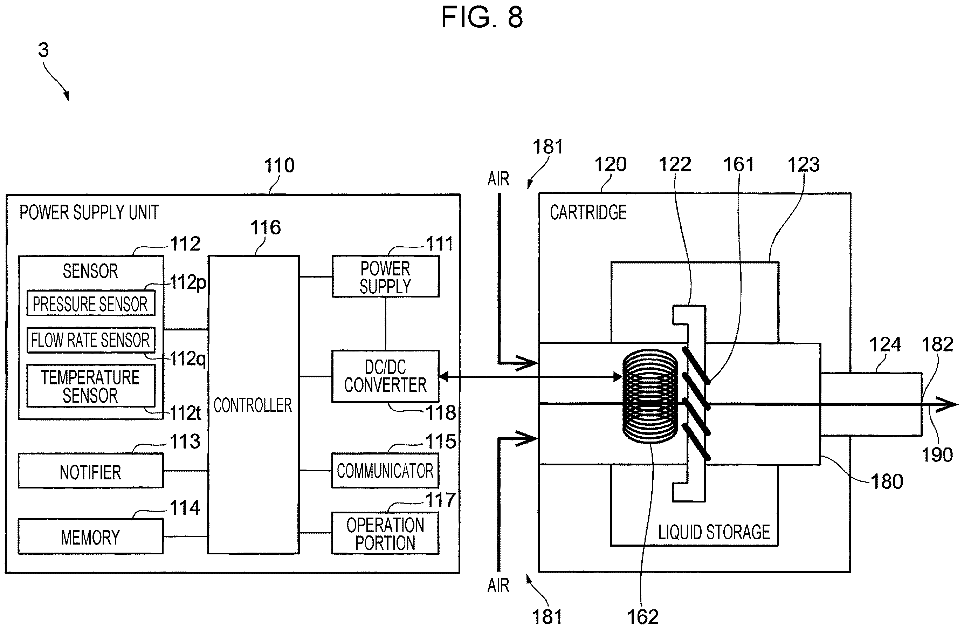

Fig. 8 is a diagram schematically illustrating an example of a schematic configuration of an inhalation device 3 according to a third embodiment.

The inhalation device 3 according to the third embodiment is different from the inhalation device 1 according to the first embodiment in that a susceptor 161 and an electromagnetic induction source 162 are provided instead of the heater 121. Hereinafter, differences from the first embodiment will be described. In the first embodiment and the third embodiment, the same items are denoted by the same reference numerals, and detailed description thereof will be omitted.

The susceptor 161 produces heat by electromagnetic induction. The susceptor 161 is formed of a conductive material such as metal. The susceptor 161 is disposed close to the liquid guide 122. In the example illustrated in Fig. 8, the susceptor 161 is formed of a metal conductive wire and is wound around the liquid guide 122.

The electromagnetic induction source 162 causes the susceptor 161 to produce heat by electromagnetic induction. The electromagnetic induction source 162 is formed of, for example, a coiled conductive wire. The electromagnetic induction source 162 generates a magnetic field when an alternating current is supplied from the power supply 111. The electromagnetic induction source 162 is disposed at a position where the susceptor 161 overlaps with the generated magnetic field. Therefore, when the magnetic field is generated, an eddy current is generated in the susceptor 161, and Joule heat is generated. Then, the aerosol source held in the liquid guide 122 is heated and atomized by the Joule heat, and aerosol is generated.

In the inhalation device 3 according to the third embodiment configured as described above, the controller 116 controls the electric power supply to the electromagnetic induction source 162 in the same manner as controlling the electric power supply to the heater 121 according to the first embodiment to perform a heating process of the susceptor 161. In the heating process of the susceptor 161, the controller 116 performs the preliminary heating by the same method as that described in the first embodiment, and thus, it is possible to increase the amount of aerosol at the initial stage of inhalation and also to suppress wasteful electric power consumption associated with the preliminary heating.

### <Fourth Embodiment>

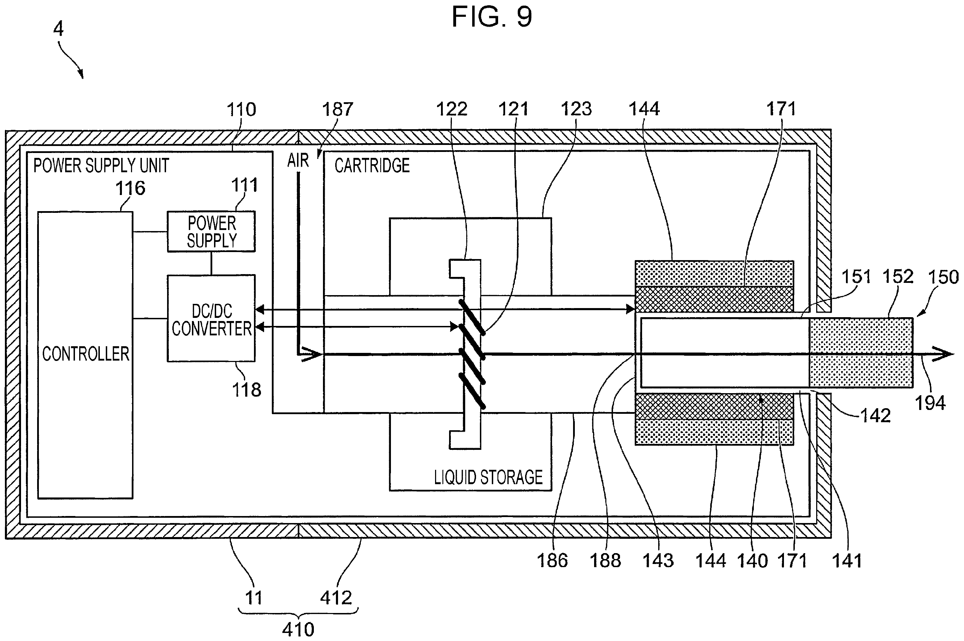

Fig. 9 is a diagram schematically illustrating an example of a configuration of an inhalation device 4 according to a fourth embodiment.

The inhalation device 4 according to the fourth embodiment is different from the inhalation device 1 according to the first embodiment in that aerosol is generated by heating an aerosol source as a liquid and heating a substrate including the aerosol source. In addition, the inhalation device 4 is different from the inhalation device 1 in that a case 410 is provided instead of the case 10. Hereinafter, differences from the first embodiment will be described. In the first embodiment and the fourth embodiment, the same items are denoted by the same reference numerals, and detailed description thereof will be omitted.

The inhalation device 4 according to the fourth embodiment includes, in addition to the power supply unit 110, the heater 121, the liquid guide 122, and the liquid storage 123, a substrate heater 171, a holder 140, and a heat insulator 144. In the inhalation device 4, the user inhales in a state in which a stick substrate 150 is held by the holder 140.

The holder 140 has an internal space 141, and holds the stick substrate 150 while partially accommodating the stick substrate 150 in the internal space 141. The holder 140 has an opening 142 that allows the internal space 141 to communicate with outside and accommodates the stick substrate 150 inserted into the internal space 141 through the opening 142. For example, the holder 140 may be a tubular body having the opening 142 and a bottom 143 on the bottom, and may define the pillar-shaped internal space 141. The holder 140 is configured such that the inside diameter is smaller than the outer diameter of the stick substrate 150 in at least a portion in the height direction of the tubular body, and can hold the stick substrate 150 so as to press the stick substrate 150 inserted into the internal space 141 from the outer circumference. The holder 140 also has a function of defining an airflow path passing through the stick substrate 150. An air inlet hole that is an inlet of air into the flow path is disposed, for example, in the bottom 143. Meanwhile, the opening 142 serves as an air outlet hole that is an outlet of air from the flow path.

The stick substrate 150 is a stick member. The stick substrate 150 includes a substrate 151 and an inhalation port 152.

The substrate 151 includes an aerosol source. The aerosol source is heated and atomized to produce aerosol. The aerosol source may be derived from tobacco, such as shredded tobacco or a processed product of a tobacco raw material formed into a granular, sheet-like, or powdery from. The aerosol source may not be derived from tobacco and may be made from a plant other than tobacco (such as mint and herb). As an example, the aerosol source may include a flavor component such as menthol. In a case where the inhalation device 4 is a medical inhaler, the aerosol source may include medicine for a patient to inhale. Note that the aerosol source is not limited to a solid and may be, for example, polyhydric alcohols such as glycerin and propylene glycol, and liquids such as water. At least a part of the substrate 151 is accommodated in the internal space 141 of the holder 140 in a state in which the stick substrate 150 is held by the holder 140.

The inhalation port 152 is a portion to be held by the user in the mouth during inhalation. At least a part of the inhalation port 152 protrudes from the opening 142 in a state in which the stick substrate 150 is held by the holder 140. When the user holds the inhalation port 152 protruding from the opening 142 in the mouth and inhales, air is introduced into the holder 140 through an air inlet hole 187. The introduced air passes through the internal space 141 of the holder 140, that is, the substrate 151, and reaches the inside of the user's mouth together with aerosol generated from the substrate 151.

The substrate heater 171 heats the substrate 151 to atomize the aerosol source and generate aerosol. The substrate heater 171 is formed of any material such as metal or polyimide. For example, the substrate heater 171 has a film-like shape and surrounds the outer circumference of the holder 140. When the substrate heater 171 produces heat, the aerosol source contained in the stick substrate 150 is heated from the outer circumference of the stick substrate 150 and atomized to generate aerosol. The substrate heater 171 produces heat when receiving electric power from the power supply 111.

Here, an air outlet hole 188 of an airflow path 186 is disposed in the bottom 143 of the holder 140. The internal space 141 of the holder 140 and the airflow path 186 communicate with each other via the air outlet hole 188.

The airflow path 186 is a flow path of air to be inhaled by the user. The airflow path 186 has a tubular structure in which the air inlet hole 187, which is an inlet of air into the airflow path 186, and the air outlet hole 188, which is an outlet of air from the airflow path 186, are at both ends. As the user inhales, air is introduced into the airflow path 186 through the air inlet hole 187, and air flows out of the internal space 141 of the holder 140 through the air outlet hole 188. As an example, the air inlet hole 187 is disposed at any position of the inhalation device 4. On the other hand, the air outlet hole 188 is disposed in the bottom 143 of the holder 140. The liquid guide 122 is disposed in the middle of the airflow path 186. The aerosol generated by the heater 121 is mixed with air introduced through the air inlet hole 187. Subsequently, as the user inhales, the mixture fluid of the aerosol and the air is conveyed to the internal space 141 of the holder 140 via the air outlet hole 188 as indicated by an arrow 194. Then, the mixture fluid of the aerosol and the air conveyed to the internal space 141 of the holder 140 reaches the inside of the user's mouth together with the aerosol generated by the substrate heater 171.

The case 410 includes the power supply unit case 11, and a cylindrical heater case 412 that accommodates the heater 121, the liquid guide 122, the liquid storage 123, the holder 140, the substrate heater 171, the heat insulator 144, and the like. As an example, the power supply unit case 11 and the heater case 412 may be configured to be separate bodies and detachable from each other. However, the power supply unit case 11 and the heater case 412 may be integrally formed.

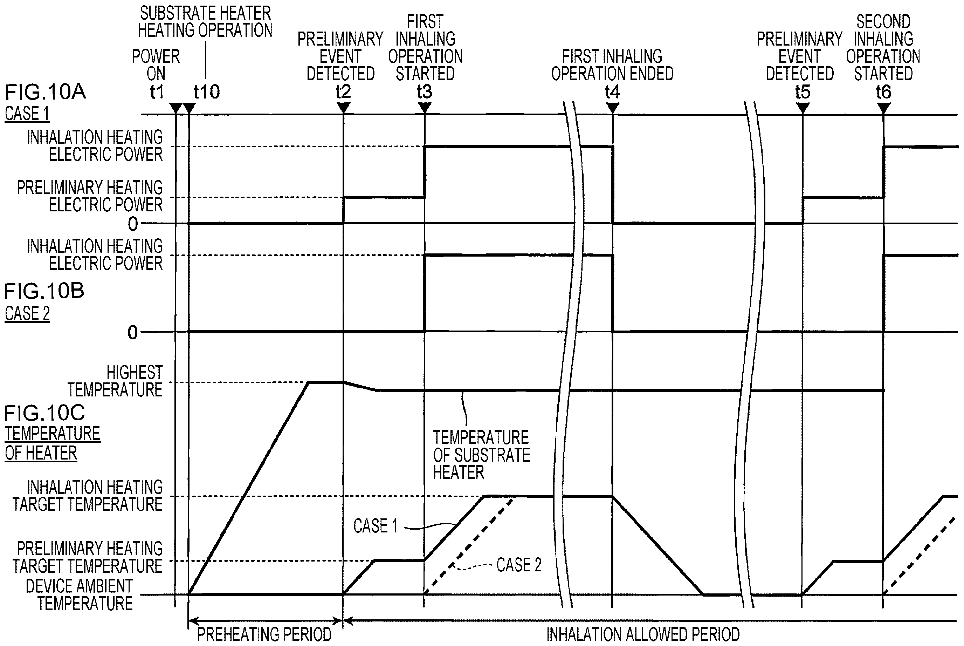

Figs. 10A to 10C are timing charts for describing operations of the inhalation device 4.

In the inhalation device 4 according to the fourth embodiment configured as described above, after the inhalation device 4 is powered on and activated, upon an operation for starting heating of the substrate heater 171 (hereinafter, may be referred to as a "substrate heater heating operation") being performed on the operation portion 117 at time t10, the controller 116 starts electric power supply to the substrate heater 171 to start the heating of the substrate heater 171. As an example, the substrate heater heating operation may be pressing the operation portion 117 for a long time of 2 seconds or more. Subsequently, the controller 116 controls the electric power supplied to the substrate heater 171 via the DC/DC converter 118 so as to implement time-series transition of the target temperature defined in a heating profile stored in the memory 114 in advance. For example, the controller 116 controls the electric power supplied to the substrate heater 171, based on the deviation between the target temperature defined in the heating profile and the actual temperature of the substrate heater 171 (hereinafter, may be referred to as "actual temperature"). The temperature control of the substrate heater 171 can be implemented by, for example, known feedback control.

A period from the start of the heating of the substrate heater 171 to the start of a period in which the user is allowed to perform the inhaling operation may be referred to as a "preheating period", and a period in which the preheating period ends and the stick substrate 150 can generate a sufficient amount of aerosol may be referred to as an "inhalation allowed period". The preheating period ends after the temperature of the substrate heater 171 reaches a highest temperature (for example, 295 degrees) determined in advance. As an example, the preheating period may end when a time (for example, 10 seconds) determined in advance elapses after the temperature of the substrate heater 171 reaches the highest temperature (for example, 295 degrees) determined in advance. Alternatively, as an example, the preheating period may end when a time (for example, 30 seconds) determined in advance elapses after the heating of the substrate heater 171 is started. When the preheating period ends and the inhalation allowed period starts, the controller 116 notifies the user via the notifier 113 that the inhalation allowed period has started. During the inhalation allowed period, the temperature of the substrate heater 171 is maintained within a temperature range (for example, 230 degrees to 295 degrees) determined in advance.

In the inhalation device 4 according to the fourth embodiment configured as described above, during the inhalation allowed period, the controller 116 performs the preliminary heating of the heater 121 by the same method as that described in the first embodiment, and thus, it is possible to increase the amount of aerosol at the initial stage of inhalation and also to suppress wasteful electric power consumption associated with the preliminary heating.

Then, the controller 116 of the inhalation device 4 may determine the preliminary event when the preheating period ends and the inhalation allowed period starts. That is, the controller 116 may start the preliminary heating of the heater 121 when the preheating period ends and the inhalation allowed period starts. Accordingly, it is possible to suppress wasteful electric power consumption associated with the preliminary heating with high accuracy.

Like the inhalation device 1, the inhalation device 4 may include at least one of the gyro sensor 112j, the tactile sensor 112s, the acceleration sensor 112a, the height sensor 112h, the LiDAR 1121, the infrared sensor 112i, the camera 112c, the odor sensor 112n, the tactile sensor 112m, the humidity sensor 112k, and the CO₂ sensor 112o, and the controller 116 may detect the preliminary event or determine that the preliminary heating end condition is satisfied, based on the output value from one sensor or the like. As an example, the LiDAR 1121, the infrared sensor 112i, the camera 112c, the odor sensor 112n, the humidity sensor 112k, and the CO₂ sensor 112o may be attached to the heater case 412. By being attached to the heater case 412, the magnitude of the distance between the inhalation device 4 and the mouth can be grasped more accurately than in a case of being attached to the power supply unit case 11.

### Reference Signs List

- 1, 2, 3, 4: inhalation device
- 10: case
- 11: power supply unit case
- 12: cartridge case
- 20: end cap
- 110: power supply unit
- 111: power supply
- 112: sensor
- 112a: acceleration sensor
- 112c: camera
- 112h: height sensor
- 112i: infrared sensor
- 112j: gyro sensor
- 112k: humidity sensor
- 1121: LiDAR
- 112m: tactile sensor
- 112n: odor sensor
- 112o: CO₂ sensor
- 112p: pressure sensor
- 112q: flow rate sensor
- 112s: tactile sensor
- 112t: temperature sensor
- 116: controller
- 117: operation portion
- 118: DC/DC converter
- 120: cartridge
- 121: heater
- 122: liquid guide
- 123: liquid storage
- 124: mouthpiece

## Claims

1. An inhalation device comprising:
a liquid storage configured to store a liquid for generating aerosol by being heated;
a heater configured to heat the liquid;
a power supply configured to accumulate electric power; and
a controller configured to control electric power supply from the power supply to the heater, wherein
if a predetermined condition determined in advance is satisfied, the controller controls the electric power supply to perform first heating in which a temperature of the liquid becomes higher than or equal to a first temperature at which the liquid is vaporized, and, before the predetermined condition is satisfied, the controller performs, by performing the electric power supply, second heating in which the temperature of the liquid becomes a temperature higher than or equal to a second temperature and lower than the first temperature, and then, if an end condition determined in advance is satisfied, the controller stops the second heating.

2. The inhalation device according to claim 1, wherein
the end condition is satisfied if a time determined in advance elapses after the second heating is started.

3. The inhalation device according to claim 1, comprising:
a gyro sensor, wherein
the end condition is satisfied if an output value of the gyro sensor changes, from a value indicating that a height of an inhalation port to be held in a mouth at a time of inhalation of the aerosol is higher than a height of the power supply, to a value indicating that the power supply and the inhalation port are at the same height.

4. The inhalation device according to claim 1, comprising:
an acceleration sensor, wherein
the end condition is satisfied if an output value of the acceleration sensor becomes smaller than or equal to a negative threshold value determined in advance.

5. The inhalation device according to claim 1, comprising:
a tactile sensor, wherein
the end condition is satisfied if an output value of the tactile sensor does not indicate a value indicating a contact of a hand of a user.

6. The inhalation device according to claim 1, wherein
the end condition is satisfied if a distance to a mouth of a user exceeds a threshold value determined in advance.

7. The inhalation device according to claim 6, comprising:
at least one device of a LiDAR configured to measure the distance, an infrared sensor configured to measure a body temperature of the user, a camera, an odor sensor, a tactile sensor, a humidity sensor, and a CO₂ sensor, wherein
the controller grasps that the distance exceeds the threshold value by using an output value of the device.

8. The inhalation device according to any one of claims 1 to 7, wherein
the controller sets a value of the electric power at a time of the second heating to be smaller than a value of the electric power at a time of the first heating.

9. The inhalation device according to any one of claims 1 to 8, wherein
the controller performs control such that a temperature of the heater does not exceed a target temperature.
